**Europäisches Patentamt**

**European Patent Office**

**Office européen des brevets**

(11) Publication number: **0 119 062**
**B1**

## EUROPEAN PATENT SPECIFICATION

(45) Date of publication of patent specification: **27.09.89**

(21) Application number: **84301544.7**

(22) Date of filing: **08.03.84**

(51) Int. Cl.⁴: **A 61 K 6/06**

(54) Storable dental porcelain paste.

(30) Priority: **09.03.83 US 473468**

(43) Date of publication of application:
**19.09.84 Bulletin 84/38**

(45) Publication of the grant of the patent:
**27.09.89 Bulletin 89/39**

(84) Designated Contracting States:
**AT BE CH DE FR GB IT LI NL SE**

(56) References cited:
**US-A-4 101 330**

(73) Proprietor: **JOHNSON & JOHNSON DENTAL PRODUCTS COMPANY**
**20 Lake Drive CN 7060**
**East Windsor New Jersey 08520 (US)**

(72) Inventor: **Panzera, Carlino**
**7 Huntsman Lane**
**Belle Mead NJ 08502 (US)**
Inventor: **Jones, Robin Mackay Forbes**
**45C Pennington-Titusville Road**
**Titusville NJ 08500 (US)**

(74) Representative: **Jones, Alan John et al**
**CARPMAELS & RANSFORD 43 Bloomsbury Square**
**London, WC1A 2RA (GB)**

Courier Press, Leamington Spa, England.

## Description

The invention relates to dental porcelain in a storable, paste form.

Dental porcelain that is employed in the production of prostheses such as crowns and bridges, is typically marketed in the form of a fine powder. When the powder is to be used, it is then mixed with a liquid, usually water containing a small amount of a wetting agent, and is then applied to a metallic coping as a thin film.

The practice of mixing the powder with liquid just before use necessarily entails a certain amount of waste, because more material will ordinarily be mixed then will be used. It would be desirable to be able to premix the porcelain powder with the liquid dispersant; however, the conventional porcelain powders that are now employed in the manufacture of dental prostheses exhibit the property of dilatancy when mixed with water. This means that the water/porcelain powder mixture tends to harden upon standing, and actually tends to become stiffer under the influence of mixing or shearing forces. Thus, dilatancy is, in one sense, the opposite of the property known as "thixotropy".

The present invention is based upon the discovery that a finely divided ceramic material can be added to the dental porcelain powder in a small proportion, and that such finely divided ceramic will overcome the tendency of water/porcelain powder mixtures to become dilatant. As an added bonus, the resulting mixture of water, porcelain powder and finely divided ceramic exhibits a more plastic-like feel, is less gritty and has slightly better working properties than the conventional porcelain powder/water mixtures.

The invention provides a storable paste-like material, for use in preparing porcelain dental prostheses, which comprises porcelain powder, water and finely divided ceramic having firing properties that are compatible with those of the porcelain powder employed, said finely divided ceramic having a particle size less than 0.3 µm and being present in an amount of from 0.25 to 1 weight per cent, based on weight of porcelain powder, such that it overcomes the dilatance of the mixture of porcelain powder and water.

The invention provides also a method of preparing a storable paste-like material for use in preparing porcelain dental prostheses, comprising mixing together porcelain powder, water and finely divided ceramic having firing properties that are compatible with those of the porcelain powder employed, said finely divided ceramic having a particle size less than 0.3 µm and being present in an amount of from 0.25 to 1 weight per cent, based on weight of porcelain powder, such that it will overcome the dilatance of the mixture of porcelain powder and water.

The commercial supplier of "VEEGUM"® materials, which are colloidal magnesium aluminum silicates or magnesium silicates, recommends that they be used as suspending agents in ceramics, as plasticizing agents for nonplastic formulations, such as high alumina or zirconia bodies, and as nonmigrating binders in extruded bodies.

The dental porcelain powders that are employed in the invention are well-known materials. They are generally formed from mixtures of oxides of silicon, aluminum, titanium, alkali metals, alkaline earth metals and boron. The mixture of oxides is selected to possess certain properties, such as to match the coefficient of thermal expansion of the metal copings on which the porcelain will be coated, as well as the appropriate mixture of opacity and translucency so that the porcelain will resemble as closely as possible the natural tooth. All of these factors are well-known in the art, and do not constitute part of this invention.

The porcelain powders are usually finely divided, having average particle sizes in the range of from 20 to 40 µm, and more usually 30±5 µm.

The finely divided ceramic additive that is employed in the invention is used for the purpose of neutralizing or overcoming the tendency of porcelain powders to form a dilatant paste when mixed with water or other liquid. Preferred materials for use in the invention are colloidal magnesium aluminum silicate and magnesium silicate. Other ceramics can also be used in the invention, provided that they have firing properties that are compatible with those of the dental porcelain employed.

The necessary qualities of the ceramic powder are the following:

First, it should melt or dissolve in the porcelain at a temperature no higher than the porcelain's maturing temperature;

Second, it should not significantly alter the thermal expansion, corrosion resistance, and shade (color) properties of the porcelain; and

Third, it must be very finely divided, i.e. colloidal, as discussed below.

The powdered ceramic is employed in finely divided form, that is, it has an average particle size such that it will impart thixotropic properties to a mixture of porcelain powder, water and the finely divided ceramic, when the ceramic is used in small proportions. The particle size of the ceramic should be less than 0.3 µm, and preferably it should have an average particle size of less than 0.1 µm.

The powdered ceramic is employed in small proportions, from 1/4 to 1 weight per cent based upon weight of the porcelain powder.

The third component of the storable paste-like material of the invention is water. Water is usually employed in a proportion of from 24 to 28 weight per cent, based upon weight of the dental porcelain powder.

The preferred way of preparing the paste-like material of the invention is to first mix the water with the finely divided ceramic, and to then add the porcelain powder. However, if desired, the ceramic can also be first mixed with the porcelain powder, followed by addition of the water.

The mixture of porcelain powder, water and finely divided ceramic provided by the invention is a paste-like material having the approximate consistency and handling characteristics of modelling clay. This material can be premixed by the manufacturer, and can be stored in tubes or syringes for ease of use by the dentist or dental technician who will be employing the material in the production of dental prostheses.

Example 1 and Control Example 1

Samples of paste are made from the following formulations:

The porcelain powder used in all the experiments has the following composition:

| Component | Weight per cent |
| --- | --- |
| $SiO_2$ | 66.7 |
| $Al_2O_3$ | 14.3 |
| MgO | 0.1 |
| CaO | 1.4 |
| $Na_2O$ | 6.0 |
| $K_2O$ | 10.0 |
| $Li_2O$ | 1.1 |
| $SnO_2$ | 0.3 |
| $ZrO_2$ | 0.1 |

The porcelain powder has the following particle size analysis:

| Particle diameter, μm. | Cumulative %, smaller |
| --- | --- |
| 5 | 7 |
| 10 | 21 |
| 20 | 48 |
| 50 | 92 |

As a Control Example, the above-described porcelain powder is mixed with distilled water containing 1/2 weight per cent of 35 per cent aqueous phosphoric acid (as a pH adjustor) and 1 weight per cent wetting agent, in proportions of 74 weight per cent powder to 26 weight per cent water. The resulting paste cannot conveniently be dispensed through a syringe opening or from a flexible tube, because the paste tends to harden when pressure is applied to it. When one attempts to squeeze it out of a flexible tube, there is a tendency for the water and powder to separate so that only water is squeezed out of the tube.

If the foregoing experiment is repeated except that 1 weight per cent of colloidal (particle size less than about 0.3 μm) magnesium silicate is added to the water (percentage based on weight of water-based on weight of porcelain powder, the percentage would be 1/3) before it is mixed with the porcelain powder, then the resulting paste is smooth, has the consistency of modelling clay, and can be stored in and squeezed out of a flexible tube or a syringe having a 1/8- to 1/4-inch (.32 to .64 cm) opening. The paste also feels less gritty than the Control, and is easier to smooth on the metallic coping used for a dental restoration. After firing, the porcelain containing the colloidal ceramic has the same appearance as the fired Control porcelain when used as a thin film or layer on a dental restoration. The magnesium silicate employed is a commercially available material sold as "VEEGUM T"®. It has the following typical analysis (expressed as oxides):

| | |
| --- | --- |
| $SiO_2$ | 53.74 |
| MgO | 23.82 |
| $Al_2O_3$ | 0.97 |
| $Fe_2O_3$ | 0.15 |
| CaO | 4.54 |
| $Na_2O$ | 1.74 |
| $K_2O$ | 0.1 |
| $Li_2O$ | 0.98 |
| Ignition Loss | 11.81 |

**Claims**

1. A storable paste-like material, for use in preparing porcelain dental prostheses, which comprises porcelain powder, water and finely divided ceramic having firing properties that are compatible with those of the porcelain powder employed, said finely divided ceramic having a particle size less than 0.3 μm and being present in an amount of from 0.25 to 1 weight per cent, based on weight of porcelain powder, such that it overcomes the dilatance of the mixture of porcelain powder and water.

2. The storable paste-like material of claim 1 wherein said finely divided ceramic is a magnesium silicate.

3. The storable paste-like material of claim 1 or claim 2 which contains from 24 to 28 weight per cent, based on weight of porcalain powder, of water.

4. A method of preparing a storable paste-like material for use in preparing porcelain dental prostheses, comprising mixing together porcelain powder, water and finely divided ceramic having firing properties that are compatible with those of the porcelain powder employed, said finely divided ceramic having a particle size less than 0.3 μm and being present in an amount of from 0.25 to 1 weight per cent, based on weight of porcelain powder, such that it will overcome the dilatance of the mixture of porcelain powder and water.

5. The method of claim 4, wherein the water is first mixed with the finely divided ceramic and the porcelain powder is then added.

6. The method of claim 4 or claim 5, wherein the amount of water is from 24 to 28 weight per cent, based on weight of porcelain powder.

**Patentansprüche**

1. Lagerfähiges pastenartiges Material, das bei der Herstellung von Porzellan-Zahnprothesen verwendbar ist, welches Material Porzellanpulver, Wasser und fein verteiltes keramisches Material enthält, dessen Brenneigenschaften mit jenen des eingesetzten Porzellanpulvers verträglich sind, wobei das fein verteilte keramische Material eine Teilchengröße unter 0,3 µm besitzt und in einem Anteil von 0,25 bis 1 Gew.%, bezogen auf das Gewicht des Porzellanpulvers, vorhanden ist, so daß es der Dilatanz der Mischung von Porzellanpulver und Wasser entgegenwirkt.

2. Lagerfähiges pastenartiges Material nach Anspruch 1, bei dem das fein verteilte keramische Material ein Magnesiumsilikat ist.

3. Lagerfähiges pastenartiges Material nach Anspruch 1 oder 2, das 24 bis 28 Gew.% Wasser, bezogen auf das Gewicht des Porzellanpulvers enthält.

4. Verfahren zur Herstellung eines lagerfähigen pastenartigen Materials, das bei der Herstellung von Porzellan-Zahnprothesen verwendbar ist, wobei Porzellanpulver, Wasser und fein verteiltes keramisches Material, dessen Brenneigenschaften mit jenen des Porzellanpulvers verträglich sind, gemischt werden, wobei das fein verteilte keramische Material eine Teilchengröße unter 0,3 µm aufweist und in einem Anteil von 0,25 bis 1 Gew.%, bezogen auf das Gewicht des Porzellanpulvers vorhanden ist, so daß es der Dilatanz der Mischung aus Porzellanpulver und Wasser entgegenwirkt.

5. Verfahren nach Anspruch 4, bei dem zuerst das Wasser mit dem fein verteilten keramischen Material gemischt und dann das Porzellanpulver zugegeben wird.

6. Verfahren nach Anspruch 4 oder 5, bei dem der Anteil des Wassers 24 bis 28 Gew.% bezogen auf das Gewicht des Porzellanpulvers beträgt.

**Revendications**

1. Matériau sous forme de pâte pouvant être stocké, en vue d'une utilisation dans la préparation de prothèses dentaires en porcelaine, qui comporte de la poudre de porcelaine, de l'eau et de la céramique finement divisée possédant des propriétés de cuisson qui sont compatibles à celles de la poudre de pocelaine utilisée, ladite céramique finement divisée ayant une dimension de particule inférieure à 0,3 µm et étant présente selon une quantité comprise entre 0,25 et 1% en poids rapporté au poids de poudre de porcelaine, de telle sorte qu'elle surmonte la tendance au durcissement du mélange de poudre de porcelaine et d'eau.

2. Matériau sous forme de pâte pouvant être stocké selon la revendication 1, dans lequel ladite céramique finement divisée est un silicate de magnésium.

3. Matériau sous forme de pâte pouvant être stocké selon la revendication 1 ou la revendication 2, qui contient de 24 à 28% en poids d'eau, rapporté au poids de poudre de porcelaine.

4. Procédé de préparation d'un matériau sous forme de pâte pouvant être stocké en vue d'une utilisation dans la préparation de prothèses dentaires en porcelaine, comportant le mélange de poudre de porcelaine, d'eau et de céramique finement divisée ayant des propriétés de cuisson compatibles à celles de la poudre de porcelaine utilisée, ladite céramique finement divisée ayant une dimension de particule inférieure à 0,3 µm et étant présente selon une quantité allant de 0,25 à 1% en poids, rapporté au poids de la poudre de porcelaine, de telle sorte qu'elle surmonte la tendance au durcissement de mélange de poudre de porcelaine et d'eau.

5. Procédé selon la revendication 4, dans lequel l'eau est tout d'abord mélangée à la céramique finement divisée et la poudre de porcelaine est ensuite ajoutée.

6. Procédé selon la revendication 4 ou la revendication 5, dans lequel la quantité d'eau est de 24 à 28% en poids rapporté au poids de poudre de porcelaine.